(19) Europäisches Patentamt
European Patent Office
Office européen des brevets

(11) **EP 2 254 472 B1**

(12) **EUROPEAN PATENT SPECIFICATION**

(45) Date of publication and mention
of the grant of the patent:
**25.05.2016 Bulletin 2016/21**

(51) Int Cl.:
*A61B 5/15* *(2006.01)*       *A61M 25/06* *(2006.01)*
*A61M 5/158* *(2006.01)*

(21) Application number: **08731700.4**

(22) Date of filing: **07.03.2008**

(86) International application number:
**PCT/US2008/056256**

(87) International publication number:
**WO 2009/113999 (17.09.2009 Gazette 2009/38)**

(54) **FLASHBACK BLOOD COLLECTION NEEDLE**

FLASHBACK-BLUTENTNAHMENADEL

AIGUILLE DE PRÉLÈVEMENT SANGUIN À REFOULEMENT

(84) Designated Contracting States:
**AT BE BG CH CY CZ DE DK EE ES FI FR GB GR
HR HU IE IS IT LI LT LU LV MC MT NL NO PL PT
RO SE SI SK TR**

(43) Date of publication of application:
**01.12.2010 Bulletin 2010/48**

(73) Proprietor: **Becton Dickinson and Company
Franklin Lakes, NJ 07417 (US)**

(72) Inventors:
  • **TAN, Chee Leong Alvin
    Singapore 510225 (SG)**
  • **MOH, Jon
    Singapore 681683 (SG)**

  • **SIM, Stanley
    KULAI, JOHOR
    Malaysia 8100 (MY)**

(74) Representative: **von Kreisler Selting Werner -
Partnerschaft
von Patentanwälten und Rechtsanwälten mbB
Deichmannhaus am Dom
Bahnhofsvorplatz 1
50667 Köln (DE)**

(56) References cited:
**WO-A-2006/022716       US-A- 5 295 970
US-A1- 2003 105 414       US-A1- 2006 036 219
US-B1- 6 524 277**

EP 2 254 472 B1

**Description**

BACKGROUND OF THE INVENTION

1. Field of the Invention

The present invention relates to a device for collecting blood samples by performing venipuncture on a patient. More particularly, the present invention relates to a needle assembly for multiple sample blood collection that allows a phlebotomist to determine whether vein entry has occurred when collecting a blood sample from a patient into an evacuated blood collection tube.

2. Description of Related Art

Venipuncture is the primary method used for acquiring blood samples for laboratory testing. In performing venipuncture procedures, a phlebotomist must follow several steps simultaneously. Such steps include assessing the patient's overall physical and psychological condition so as to properly select a venipuncture site and technique. The phlebotomist must also select the proper corresponding equipment, perform the technique so as to control bleeding, and properly collect and identify fluid specimens for testing. The phlebotomist must ascertain all of these coinciding factors, as such factors may adversely affect the distension of the vein and the length of the venipuncture procedure.

Various venipuncture devices have been developed to address the above-described problems. These devices include products intended to assist the phlebotomist in confirming that vein entry has been made see e.g. United States. Patent Nos. 5,222,502 and 5,303,713. Such a device contains a needle assembly with a housing that defines a chamber therein. A single cannula pointed at both ends is affixed to the housing. The intravenous (IV) end of the cannula is adapted for penetration of a patient's vein. The non-patient end of the cannula has a sealable sleeve and is adapted for penetration of a penetrable stop positioned within an evacuated container.

Upon vein entry with the intravenous end of the cannula, blood will flow through the cannula, into the sealable sleeve and into the housing chamber, which is clear or translucent for visualization ("flashback"). Once air is vented from the flashback chamber, the blood therein is pressurized each time the sealable sleeve is pushed toward the housing chamber upon activation of an evacuated container.

Due to the length of time between vein entry and flashback, the phlebotomist may erroneously believe that satisfactory vein entry has not been achieved since there is no immediate indication of vein entry in the see-through chamber. The phlebotomist may therefore unnecessarily repeat the venipuncture procedure, requiring replacement of the evacuated container and/or the needle assembly itself. Such a repetitive process prolongs the physical and emotional discomfort endured by the patient. In such cases, a phlebotomist may use a blood collection set to provide some entry indication, and will then incur the cost of the blood collection set, as well as the cost of a discard tube.

It would therefore be desirable to provide an improved blood collection device that permits blood flow through a relatively short needle directly into a flashback chamber, thereby providing immediate indication of successful vein entry.

WO 2006/022716 A1 discloses a needle assembly including a transparent or translucent housing with a fluid inlet end, a fluid outlet end, a flashback chamber and a venting mechanism therebetween. Substantially axially aligned inlet and outlet cannulas extend from the housing and communicate with the chamber. A sealable sleeve covers the external end of the outlet cannula. The vent mechanism may contain a hydrophilic material that swells on contact with aqueous or water-containing substances.

US 2006/0036219 A1 discloses a needle assembly including a transparent or translucent housing with a fluid inlet end, a fluid outlet end, a flashback chamber and a venting mechanism therebetween. Substantially axially aligned inlet and outlet cannulas extend from the housing and communicate with the chamber. A sealable sleeve covers the external end of the outlet cannula. Relative volumes of the cannulas, the chamber and the sleeves are selected to provide rapid reliable flashback indicative of venous entry with an internal vent plug over the outlet of the flashback chamber to inhibit leakage of blood form the needle on withdrawal from the patient. Blood flow into the housing interior is prevented by the vent plug which allows the pressurized air to flow through it.

SUMMARY OF THE INVENTION

The invention provides a needle assembly for the extraction of at least one fluid sample into an evacuated container for laboratory testing. The needle assembly provides a clear or translucent housing with sufficient dead space for blood to flow into a flashback chamber for visualization by the user to confirm successful vein entry, with an internal vent mechanism.

In one embodiment, the invention relates to a needle assembly comprising a housing defining a housing interior, a cannula having a patient puncture tip extending from a first end of the housing, and a non-patient puncture tip extending from a second end of the housing. The non-patient puncture tip and the patient puncture tip are in fluid communication with each other through the cannula, such that the sole communication path between the housing interior and the external environment is via the patient puncture tip. A porous vent is positioned within the housing interior to separate the housing interior into a first chamber and a second chamber, with the cannula being in fluid communication with the first chamber. The porous vent includes pores for passage of blood therethrough from the first chamber to the second chamber. The first chamber and the second chamber are configured such that upon insertion of the patient needle tip into a patient, blood flows through the cannula and into the first chamber without sealing the porous vent. Upon application of an evacuated container to the non-patient puncture tip, blood is drawn from the first chamber and air is drawn from the second chamber, thereby establishing a negative pressure within the second chamber with respect to an external environment of the needle assembly. Blood can thereafter be drawn into the first chamber and through the porous vent, with a negative pressure maintained in the second chamber.

In one embodiment, the cannula includes a first end comprising the patient puncture tip and a second end comprising the non-patient puncture tip, with an opening between the first end and the second end providing fluid communication between the cannula and the first chamber of the housing. In an alternate embodiment, the cannula comprises a first cannula having a patient puncture tip, with the needle assembly further comprising a second cannula including the non-patient puncture tip, with the first cannula and the second cannula substantially axially aligned and separated by a gap in fluid communication with the first chamber of the housing. A sleeve may also extend about the non-patient puncture tip.

In a particular embodiment, the first end of the housing comprises an elongate longitudinal first portion having a first diameter and the second end of the housing comprises a second portion having a second diameter larger than the first diameter of the first portion. In such an embodiment, the porous vent may be positioned within the housing interior between the first portion having a first diameter and the second portion having a second diameter. Alternatively, the porous vent may be positioned within the housing interior at a location spanning the transition between the first diameter of the first position and the second diameter of the second position.

In yet a further embodiment, a method of preventing leakage of blood from a needle assembly is provided. The method involves receiving blood through a patient puncture tip and into a first chamber of a needle assembly, with the needle assembly including a needle housing defining a housing interior; a cannula having the patient puncture tip extending from a first end of the needle housing; a non-patient puncture tip extending from a second end of the needle housing, the non-patient puncture tip and the patient puncture tip being in fluid communication with each other through the cannula; and a porous vent positioned within the housing interior and separating the housing interior into a first chamber and a second chamber. The cannula is in fluid communication with the first chamber such that the sole communication path between the housing interior and the external environment is via the patient puncture tip, and the porous vent includes pores for passage of blood therethrough from the first chamber into the second chamber. Fluid communication is established between the non-patient puncture tip and an evacuated collection container, such that blood contained within the first chamber is drawn into the evacuated collection container and air is drawn out of the second chamber through the porous vent. As such, a negative pressure gradient is established within the second chamber relative to the external environment of the needle assembly, such that blood flows through the cannula into the first chamber and contacts the porous vent. Blood is then drawn through the pores of the porous vent toward the second chamber such that after removing the patient puncture tip from the vasculature of the patient any blood contained within the cannula is displaced away from the patient puncture tip based upon the negative pressure gradient established within the second chamber.

Additionally, a further step may include establishing fluid communication between the non-patient puncture tip and a second evacuated collection container prior to drawing blood through the patient puncture tip and through the cannula into the second evacuated collection container, followed by releasing the fluid communication between the non-patient puncture tip and the second evacuated collection container.

DESCRIPTION OF THE DRAWINGS

FIG. 1 is a cross-sectional view of a typical configuration of the needle assembly.

FIG. 2 is a cross-sectional view of a second configuration.

FIG. 3 is a cross-sectional view of a third configuration.

FIG. 4 is a cross-sectional view of a fourth configuration.

FIG. 5 is a schematic view of the needle assembly of FIG. 1 prior to use.

FIG. 6 is a schematic view similar to FIG. 5, but showing the first sign of venous entry.

FIG. 7 is a schematic view of a fifth configuration.

FIG. 8 is a perspective view of a needle assembly having a flash chamber in a further configuration.

FIG. 9 is a rear perspective view of the needle assembly having a flash chamber of FIG. 8.

FIG. 10 is an exploded view of the needle assembly having a flash chamber of FIG. 8.

FIG. 11A is a cross-sectional view of the needle assembly having a flash chamber of **FIG. 8.**

**FIG. 11B** is an enlarged cross-sectional view of a portion of the needle assembly of **FIG. 11A.**

**FIG. 12A** is a cross-sectional view of a needle assembly having a flash chamber used in connection with a blood collection assembly in yet a further configuration.

**FIG. 12B** is an enlarged sectional view of a portion of the needle assembly of **FIG. 12A.**

DETAILED DESCRIPTION

The invention provides a needle assembly for blood collection that provides a visual indication of vein entry ("flashback") upon collection of a blood or other fluid sample from a patient into one or more evacuated blood collection tubes and inhibits leakage of the blood or fluid sample from the IV cannula on removal from the patient.

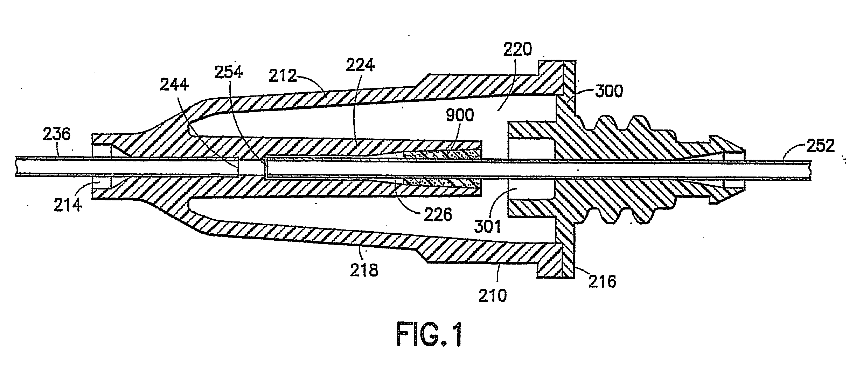

Various configurations are shown in **FIGS. 1-7.** With reference to **FIG. 1,** this configuration is directed to a needle assembly **210** with a housing **212** having a fluid inlet end **214,** a fluid outlet end **216** and a frustum-shaped exterior wall **218** extending between the ends. Exterior wall **218** defines the housing interior **220.** Housing **212** further includes a cylindrical interior wall **224** that extends in the housing interior **220** from fluid inlet end **214** substantially concentrically with cylindrical exterior wall **218** to a vent plug **900.** Cylindrical interior wall **224** and vent plug **900** define a flashback chamber **226.**

Needle assembly **210** also includes a fluid inlet cannula **236** having an exterior end that defines a sharpened bevel and an interior end **244** that is mounted fixedly in fluid inlet end **214** of housing **212.** Fluid inlet cannula **236** is characterized further by a substantially cylindrical lumen extending between the ends and communicating with the interior of housing **212.**

Needle assembly **210** further includes a fluid outlet cannula **252.** Outlet cannula **252** concludes a blunt interior end **254,** an exterior end defining a sharpened bevel and a substantially cylindrical lumen extending between the ends. Portions of outlet cannula **252** between the ends are securely affixed in outlet end **216** of housing **212.** Outlet cannula **252** is mounted so that interior end **254** passes substantially coaxially into interior wall **224** and so that interior end **254** of outlet cannula **252** substantially aligns axially with interior end **244** of inlet cannula **236.** Additionally, interior end **254** of outlet cannula **252** is spaced only a small distance from interior end **244** of inlet cannula **236.** An axial gap between interior end **254** of outlet cannula **252** and interior end **244** of inlet cannula **236** that is less than 0.5mm may result in a flashback that is inconsistent.

Cylindrical interior wall **224** is dimensioned relative to outlet cannula **252** to achieve both desirable flow of blood through assembly **210** and to achieve effective flashback indication. In particular, cylindrical interior wall **224** preferably is dimensioned to provide a radial gap around outlet cannula **252** of about 0.2mm, as indicated in **FIG. 1.** This gap achieves a substantially laminar blood flow within flashback chamber **226** and prevents blood hemolysis. Additionally, the small radial gap between cylindrical inner wall **224** and outlet cannula **252** enables a drop of blood to be spread thinly across the radial gap in flashback chamber **226** to provide a magnified flashback indication with a very small volume of blood. Thus, an easily visualized flashback indication is achieved quickly at the first appearance of blood from interior end **244** of inlet cannula **236.**

Needle assembly **210** further includes a sealable sleeve **261** mounted to fluid outlet end **216** of housing **212** and covering exterior end of outlet cannula **252** when sealable sleeve **261** is in an unbiased condition. However, sealable sleeve **261** can be collapsed in response to pressure exerted by the stopper of an evacuated tube for urging exterior end **260** of outlet cannula **252** through both sealable sleeve **261** and stopper of an evacuated tube, as known in the art.

The above configuration is described in terms of a vent plug. However, any vent mechanism is suitable. The vent mechanism may be, for example, a porous vent plug formed from a matrix or carrier material, typically hydrophobic, that is coated with, impregnated with, or otherwise, contains a hydrophilic material that swells on contact with aqueous or water containing substances. The hydrophobic carrier material can be but is not limited too, high-density polyethylene, polytetrafluoroethylene, ultra-high molecular weight polyethylene, Nylon 6, polypropylene, polyvinylidine fluoride and polyethersulfone. The swellable nature of the hydrophilic material thereby provides the sealing function in the vent upon contact with blood. It is also possible to use a porous vent plug that becomes sealed upon contact with blood using biological phenomena, e.g., by clotting and/or cell agglutination that blocks the vent; a superabsorbant material to seal the vent by swelling on contact with an aqueous fluid; or a one-way valve, (e.g., a thin flap such as plastic film covering a vent, a deformable seal such as a rubber or plastic duckbill valve, or a deformable wrap over a vent). It should be noted that any combination of these various mechanisms is also possible.

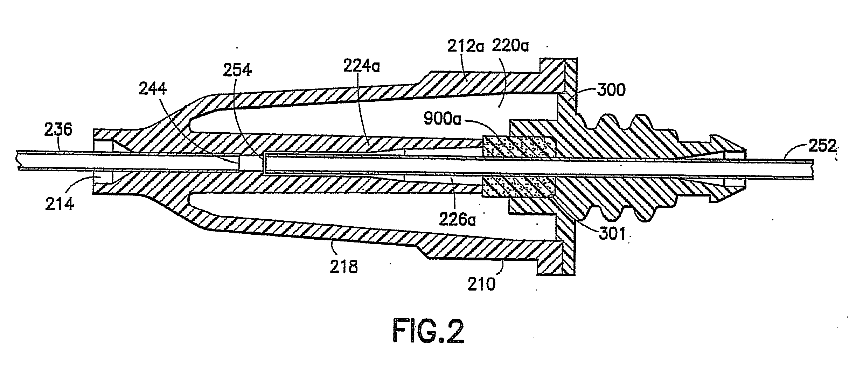

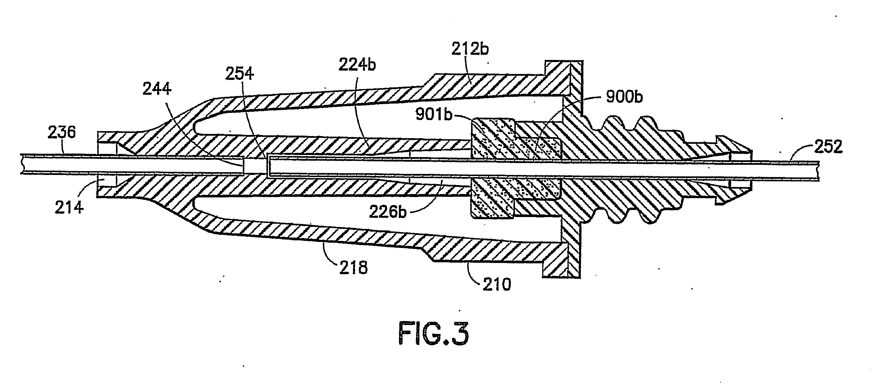

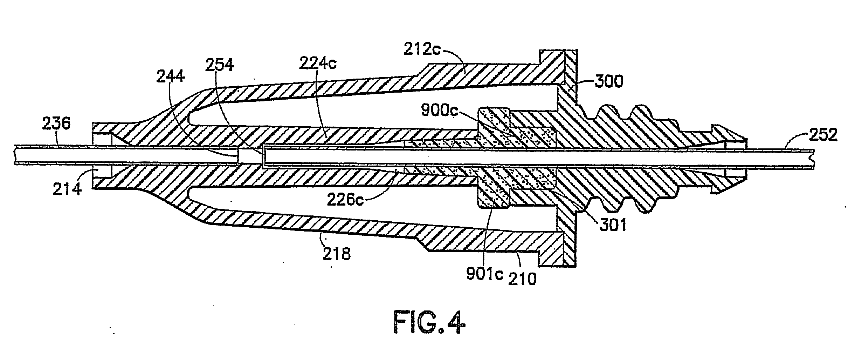

**FIGS 2-4** show configurations with varying vent plugs. **FIG. 2** shows a vent plug **900a,** which is located at the end of the cylindrical inner wall **224a** and fitted into a recess **301** in the housing interior non-patient wall **300.** **FIG. 3** shows a vent plug in a similar location to that of **FIG. 2,** however, vent plug 900b has a shoulder **901b.** **FIG. 4** shows a vent plug **900c** that is located both within the cylindrical inner wall **224c** and the recess **301** in the housing interior non-patient wall **300,** and has a shoulder **901c.** The vent plug location in each of these configurations is such that no air can flow out of the flashback chamber **226** into the housing interior **220** without passing through the vent mechanism **(900 a, b,** c).

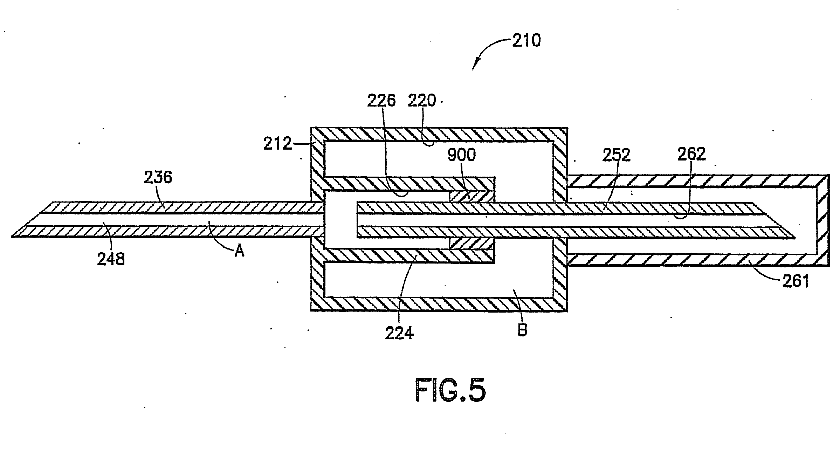

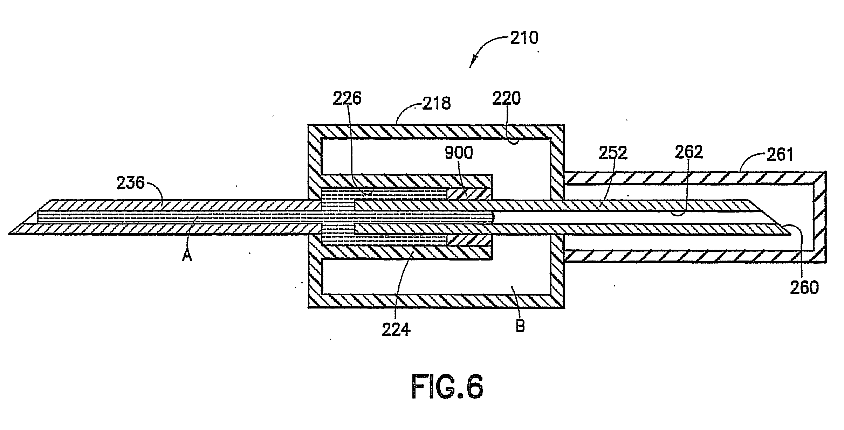

**FIGS. 5** and **6** provide schematic representations of the needle assembly **210** of **FIG. 1** before and after a conventional venipuncture, in which, the needle assembly **210** is connected to a holder (not shown) and punctures the patient's skin to make a vein entry. Upon vein entry, blood enters the IV cannula **236** and flows toward the flashback chamber **226**. The blood flows from inlet cannula **236** into the space between inlet and outlet cannula, such that blood flows both into the outlet cannula **252** and into flashback chamber **226**. At this point in time, flashback chamber **226** indicates successful vein entry and reduces the volume of air present in housing **212** shown in **FIG. 6.** Air that was at atmospheric pressure within the lumen of the IV cannula **248,** flashback chamber **226,** housing interior **220,** and the lumen of the non-patient cannula **262** prior to vein entry, thus experiences compression due to the influence of venous pressure and this air is therefore forced through the IV cannula **236** shown in **FIG. 6.** into the flashback chamber **226** and through the vent plug into chamber **220**. Blood flow into housing interior **220** is prevented by the vent plug **900,** which allows the pressurized air to flow through it, but seals on contact with blood, thereby trapping the compressed air (at venous pressure) in housing interior **220.** Blood flow in the entire needle assembly ceases once the pressure within chamber **226** and the venous pressure are equal.

Once the steps set forth in the previous paragraph occur, and venous entry is visually confirmed by the phlebotomist, an evacuated container (not shown), is then inserted into the holder such that exterior end **260** of second cannula **252** penetrates the stopper of the container, as known in the art. Upon penetration of the stopper by second cannula **252,** a negative pressure gradient is transmitted to chamber **226,** causing blood to flow from chamber **226** into the container.

The needle assemblies described above desirably should be small for convenient use, but should be constructed to ensure reliable and rapid flashback. The occurrence of flashback in the needle assemblies described and illustrated above operate pursuant to the ideal gas law. In particular, at very low densities all gases and vapors approach ideal gas behavior and closely follow the Boyle's and Charles' laws given by:

$$P_1 \, V_1 = P_2 \, V_2$$

where:

$P_1$ denotes the pressure of air within the needle assembly before needle insertion;

$P_2$ denotes the pressure of air within the needle assembly after vein entry;

$V_1$ denotes the volume of air within the needle assembly before vein entry; and

$V_2$ denotes the volume of air within the needle assembly after vein entry.

Design parameters should keep the needle device as small as possible for easy use, while ensuring an appropriate volume as specified by the preceding equation. **FIGS. 5** and **6** provide schematic representations of the needle assembly **210** of **FIG. 1** for purposes of depicting the application of the ideal gas law. In this regard, A identifies the volume of lumen **248** through inlet cannula **236**. B denotes the total volume of the housing interior **220,** flashback chamber **226,** lumen **242** through outlet cannula, **252** and sealable sleeve **261**. Referring again to the preceding equation, $P_1$ is the pressure within needle assembly **210** before use, and hence substantially equals atmospheric pressure. Atmospheric pressure will vary slightly from time to time and from location to location. However, for purposes of this analysis, atmospheric pressure $P_1$ will be assumed to be 760mm Hg. $P_2$ in the preceding equation is the volume of the dead space in needle assembly **210** after vein entry. More particularly, after vein entry, blood will fill lumen **248** of inlet cannula **236,** thereby reducing the volume to be occupied by gas in remaining portions of needle assembly **210** and hence increasing the pressure of air in the remaining portion of needle assembly **210**. A needle assembly with dimensions approximately as shown in **FIG. 1** will have a pressure $P_2$ of about 790mm Hg at venous pressure (with tourniquet). $V_1$ in the preceding equation defines the volume of the total dead spaced in needle assembly **210** before use, and hence will equal A + **B** as shown in **FIG. 5.** $V_2$ defines the dead space in the device after vein entry, and with lumen **248** of inlet cannula **236** filled with blood. Hence, $V_2$ in the preceding equation will equal **B.** These input parameters can be employed to define a minimum desired size for the respective components of needle assembly **200** as shown in the following application of the ideal gas law equation.

$$P_1 \, V_1 = P_2 \, V_2$$

$$P_1/P_2 = V_2/ \, V_1$$

$$760/790 = B/(A+B)$$

$$0.962 = B/(A+B)$$

$$0.962(A+B) = B$$

$$0.038B = 0.962A$$

$$B = 25.3A$$

Therefore, dead space in housing **212,** outlet cannula **252** and sleeve **261** advantageously is at least 25.3 times the volume defined by lumen **248** through inlet cannula **236,** and most advantageously is about 26 times the volume of lumen **248.** However, other configurations are possible and will function as described herein.

The immediate response when an evacuated tube is placed in communication with outlet cannula **252** is to draw blood from the vein into tube (not shown). The highest-pressure gradient is always maintained between the vein and the evacuated tube. An axially aligned inlet cannula **236** and outlet cannula 252, therefore provide an unobstructed path for blood flow from the vein into evacuated tube.

When the requisite tubes are filled with blood, the needle assembly is removed from the vein. The sealed nature of the vent plug **900** inhibits the pressurized air within housing interior **220** from then moving into the flashback chamber **226** and into the inlet cannula **236,** which could promote dripping of blood from the IV cannula tip.

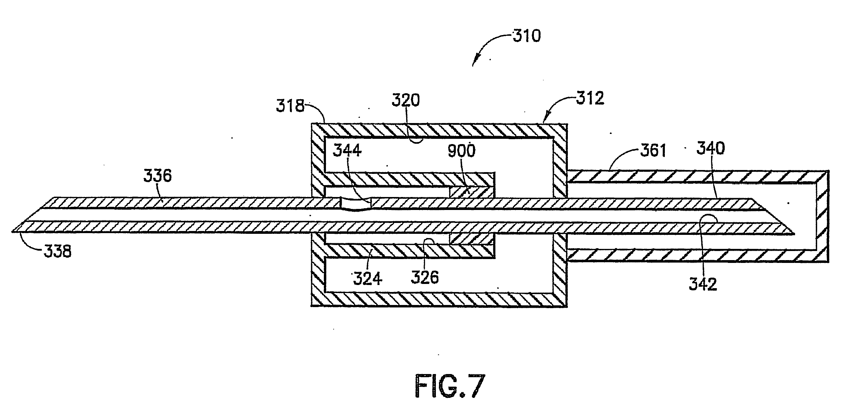

The preceding configurations show structurally separate inlet and outlet cannulas that are axially aligned with one other and placed in close end-to-end relationship with one another. However, the principals of the invention described above also can be achieved with a single cannula formed with a transverse slot or aperture within the flashback chamber. For example, **FIG. 7** schematically shows a needle assembly **310** with a housing **312** that is substantially identical to housing **212** described and illustrated above. Needle assembly **310** differs from needle assembly **210** in that a single double end needle cannula **336** is provided and passes entirely through housing **312.** More particularly, needle cannula **336** includes a venous entry end **338,** a non-patient end **340** and a lumen **342** extending therebetween. Portions of cannula 336 within inner wall 324 include a slot or aperture 344 to provide communication between lumen 342 and flashback chamber 336 within inner wall 324. Needle assembly 310 functions substantially in the same manner as needle assembly 210 described and illustrated above.

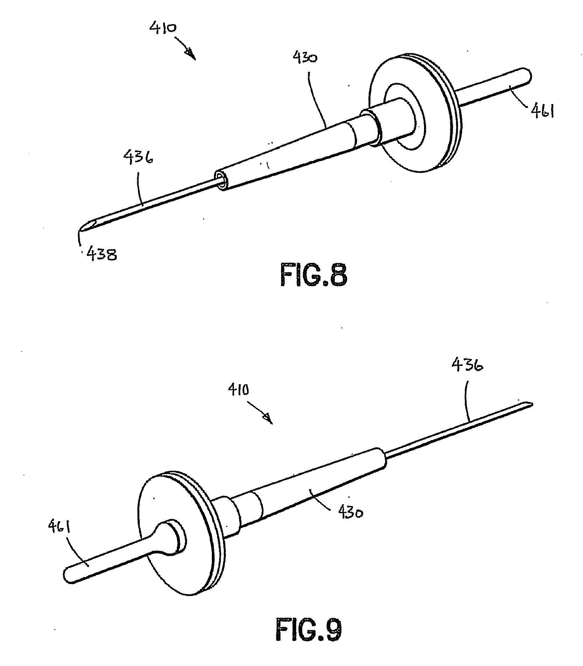

FIGS. 8-11 depict a needle assembly according to the invention. In certain embodiments of the needle assembly described with respect to FIGS. 1-7, the housing interior includes a vent plug 900, which seals the flashback chamber 226/326 from the housing interior 220/320. In such previously described embodiments, the vent plug is described as sealing upon flow of blood into the flashback chamber, thereby inhibiting any pressurized air that may build up within the housing chamber 220/320 (such as upon displacement of air from the flashback chamber 226/326 into the housing chamber 220/320 during the initial flash procedure) from moving in a reverse direction toward the inlet cannula. In the embodiment of FIGS. 8-11, a porous vent is positioned within the housing at a location such that the vent divides the housing into two chambers having sizes and dimensions to establish predetermined volumes thereto. Moreover, the porous vent remains porous to blood and does not seal upon contact with blood. Desirably the blood does not contact the porous vent at the initial flash indication, but such contact occurs at a later point during use of the assembly, as will be described in more detail herein.

For example, FIGS. 8-11 show a needle assembly 410 according to the invention which is similar to that described in connection with FIG. 1-6 above. As shown in FIGS. 8-11, needle assembly 410 includes a housing 412 having a fluid inlet end or first end 414 and a fluid outlet end or second end 416. Needle assembly 410 includes exterior wall 418 defining the housing interior. Exterior wall 418 extends generally longitudinally at the first end 414 forming an elongate longitudinal first portion 419 having a first diameter. At second end 416, exterior wall 418 forms a second portion 421 that has a second diameter that is generally larger than the first diameter of the first portion 419. Accordingly, housing 412 may form a structure having a generally T-shaped cross-section. The exterior wall 418 at second end 416 may be a separate element 428 that is attachable to main body portion 430 forming housing 412, thereby assisting in manufacture and assembly of needle assembly 410. First portion 419 and second portion 421 may be arranged relative to each other in a variety of arrangements, so long as they are capable of functioning for transport of air therebetween as discussed herein.

Needle assembly 410 further includes a fluid inlet cannula 436 extending from first end 414 of housing 412. Fluid inlet cannula 436 includes an exterior end that defines a sharpened bevel at patient puncture tip 438, and extends within first end 414 of housing 412, and may be fixedly mounted therein. Fluid inlet cannula 436 is characterized further by a substantially cylindrical lumen extending between the ends and communicating with the interior of housing 412.

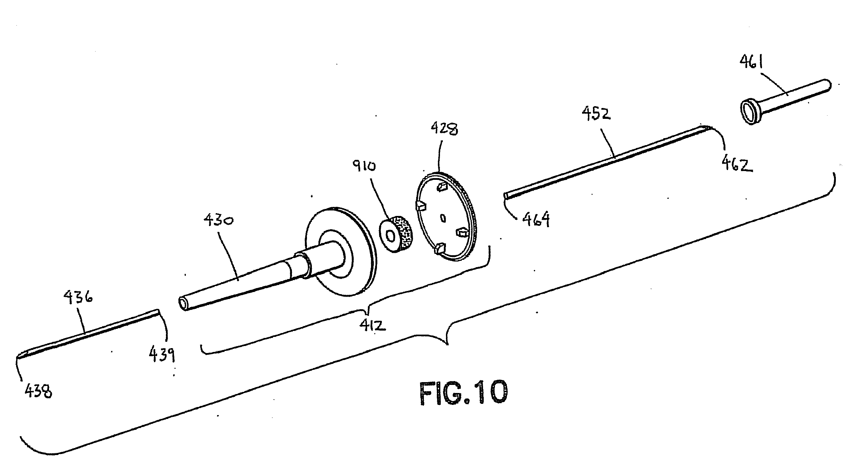

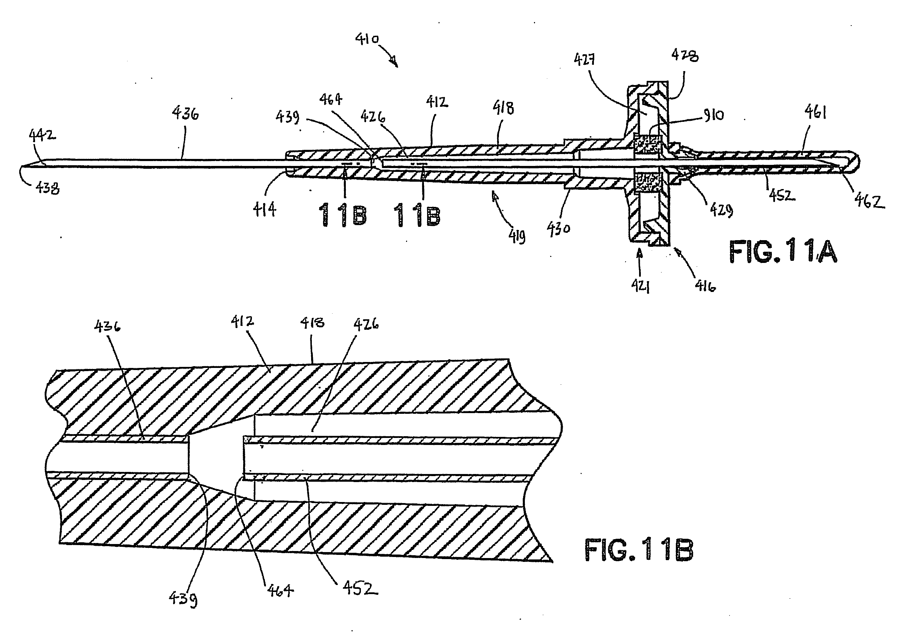

Needle assembly 410 also includes a non-patient puncture tip extending from second end 414 of housing 412. As seen in **FIG. 10,** this may be accomplished by providing needle assembly 410 with a second cannula in the form of fluid outlet cannula 452. In particular, the end of fluid outlet cannula 452 may define a sharpened bevel forming non-patient puncture tip 462. Fluid outlet cannula 452 extends within second end 416 of housing 412, and may be fixedly mounted therein. Fluid outlet cannula 452 is characterized further by a substantially cylindrical lumen communicating with the interior of housing 412. Outlet cannula 452 is mounted within housing 412 so that an interior end 464 passes substantially coaxially therein such that outlet cannula **452** substantially aligns axially with the interior end of inlet cannula **436.** Desirably, this is achieved by mounting outlet cannula **452** at a location adjacent second end **416** of housing **412,** such that the interior end **464** of outlet cannula **452** extends within housing **412** to a location adjacent the interior end of inlet cannula **436.** As seen in FIG. 11B, the interior end **464** of outlet cannula **452** is spaced only a small distance from the interior end of inlet cannula **436,** thereby forming an axial gap therebetween for flow of blood into flashback chamber **426** about outlet cannula **452.** The distance between the interior end **464** of outlet cannula **452** and the interior end of inlet cannula **436** forming the axial gap is sufficient to provide for flow of blood into flashback chamber **426** based upon the patient's blood pressure after venipuncture. In certain embodiments, an axial gap that is less than 0.5mm may result in a flashback that is inconsistent.

As seen in **FIG. 11B,** fluid inlet cannula **436** and fluid outlet cannula **452** are positioned and dimensioned within housing **412** so as to achieve both desirable flow of blood through assembly **410** and to achieve effective flashback indication. In particular, wall **418** of housing **412** is dimensioned to provide a radial gap around outlet cannula **452** of about 0.2mm at an area surrounding the internal end **464** thereof. This gap achieves a substantially laminar blood flow within flashback chamber **426** and prevents blood hemolysis.

Additionally, the small radial gap between the inner surface of wall **418** and outlet cannula **452** at the area surrounding the internal end **464** enables a drop of blood to be spread thinly across the radial gap in flashback chamber **426** to provide a magnified flashback indication with a very small volume of blood. Thus, an easily visualized flashback indication is achieved quickly at the first appearance of blood within flashback chamber **426.** It is contemplated that internal end **464** of outlet cannula **452** may be partially supported within housing **412,** so long as blood flow into flashback chamber **426** is achieved about the internal end **464.**

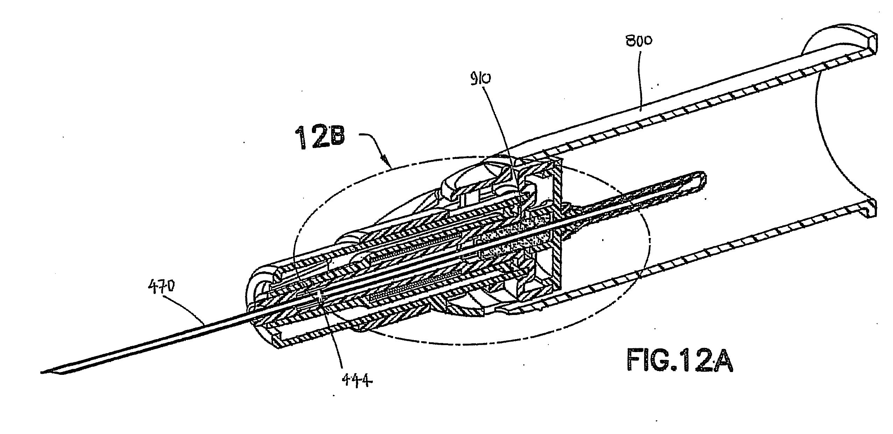

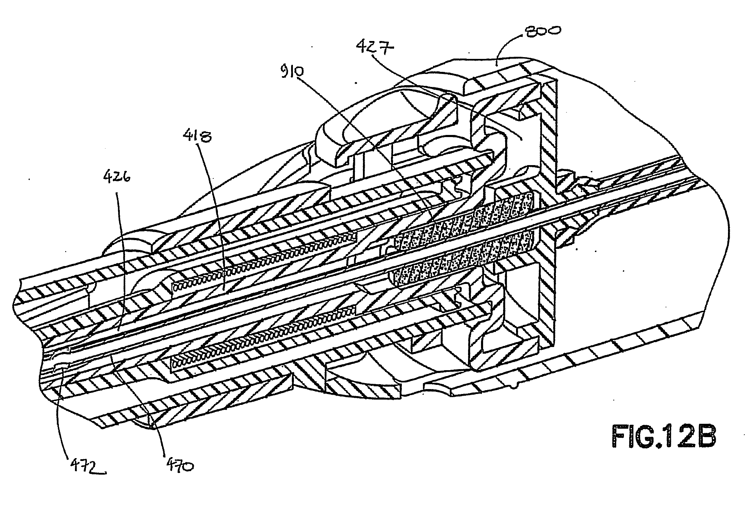

In an alternate arrangement, a single cannula is provided, similar to that embodiment discussed in connection with **FIG. 7.** Such an arrangement is depicted in the embodiment of **FIG. 12A** and **12B** (shown in connection with a blood collection assembly as will be described in more detail herein). In such an arrangement, the fluid inlet cannula and the fluid outlet cannula, represent one single cannula **470,** having a patient puncture tip **438** a non-patient puncture tip **462,** and a lumen **442** extending therethrough, and with the body of the cannula **470** being fixedly attached to a portion of the housing **412** and passing entirely through housing **412.** A portion of cannula **470** extending through housing **412** includes one or more openings such as a slot or aperture **444 to** provide communication between lumen **442** and flashback chamber **436** within housing **412.** In the embodiment seen in **FIGS. 12A** and **12B,** two separate apertures are shown on opposing sides of cannula **470,** although it is contemplated that any number of such openings can be included to provide for blood flow into flashback chamber **436.**

Returning to the embodiment of **FIGS. 8-11,** needle assembly **410** further includes a sealable sleeve **461** mounted to fluid outlet end **416** of housing **412.** This may be accomplished by providing a mounting protrusion **429** at second end **416** of housing **412,** such as on element **428,** with sealable sleeve **461** representing an elastomeric element that can be frictionally fit or otherwise affixed over protrusion **429.** Sealable sleeve **461** covers non-patient puncture tip **462** at the exterior end of outlet cannula **452** when sealable sleeve **461** is in an unbiased condition. However, sealable sleeve **461** can be collapsed in response to pressure exerted by the stopper of an evacuated tube for urging exterior end **460** of outlet cannula **452** through both sealable sleeve **461** and the stopper of an evacuated tube, as known in the art.

The embodiment of **FIGS. 8-11** further includes a porous vent **910** positioned within the interior of housing **412.** Porous vent **910** is positioned within housing **412** to divide housing **412** into two distinct chambers, namely, a first chamber represented by flashback chamber **426** and a second chamber represented by secondary chamber **427.** Porous vent **910** may be constructed of a suitable material as described above with respect to vent plug **900,** albeit without the hydrophilic material that swells on contact. In this manner, porous vent **910** is adapted to vent air therethough, and represents a porous structure including a plurality of pores that allow for passage of blood therethrough. As discussed in more detail herein, during use of needle assembly **410,** the internal pores within porous vent 910 at least partially fill with blood due to the negative pressure established within secondary chamber **427.** Such filled pores in combination with the negative pressure within secondary chamber **427** prevent air flow between the secondary chamber **427** and the flashback chamber **426,** and provide for fluid resistance of the blood flow through porous vent **910,** as will be described in further detail.

Desirably, porous vent **910** is positioned within the interior of housing **412** between first portion 419 and second portion **421.** In this manner, first portion **419** of housing **412** essentially defines the flashback chamber **426,** and second portion **421** of housing **412** essentially defines the secondary chamber **427.** Alternatively, porous vent **910** may be positioned within the interior of housing **412** at a location spanning the transition between the first diameter of first portion **419** and the second diameter of second portion **421,** as shown in the embodiment of **FIGS. 12A** and **12B.** In any event, porous vent **910** is generally a cylindrically-shaped member with a central opening therein axially encircling a portion of the cannula, particularly fluid outlet cannula **452.**

The interior volume of housing **412** is defined by the sum of the volumes of flashback chamber **426** and secondary chamber **427** as well as the volume represented by the pores of porous vent **910.** Such interior volume is configured so as to provide for certain attributes to the needle assembly **410,** in particular with respect to the ability of the secondary chamber **427** to be at least partially evacuated of a portion of the air therein to establish a negative pressure therein upon application of an evacuated tube to needle assembly **410** during use thereof. Such negative pressure within secondary chamber **427** draws blood through the pores of porous vent **910** based on when blood contacts porous vent **910** and partially fills the pores thereof. In a particular embodiment of the invention, the overall interior volume of housing 412 may be from about 300 mm$^3$ to about 400 mm$^3$. Such a volume is particularly useful for the intended use of needle assembly 410 for conventional venipuncture for drawing a blood sample from a patient using a needle cannula having a conventional gauge for venipuncture as is known in the art. With such an internal volume, porous vent 910 is desirably positioned within housing interior so as to define flashback chamber 426 as having a volume that represents from about 5 percent to about 20 percent of the total overall volume of housing 412, desirably from about 7 percent to about 12 percent of the total overall volume of housing 412, including the volume of secondary chamber 427 and the volume of the pores within porous vent 910. Such a ratio of the flashback chamber 426 to the total overall volume of the housing 412 assures that flashback chamber 426 has sufficient volume to properly visualize the initial flash, and desirably while preventing blood from fully contacting the porous vent 910 at initial venipuncture, based on the initial build-up of pressure within secondary chamber 427 caused by venous pressure forcing the blood into flashback chamber 426. Such volume ratios are effective for the intended use as described in further detail herein, wherein blood flowing into flashback chamber 426 upon initial venipuncture does not contact porous vent 910, and wherein at least a portion of the air is drawn out from secondary chamber 427 based upon application of an evacuated blood collection tube to the needle assembly 410. In this manner, secondary chamber 427 can effectively draw blood from within flashback chamber 426 and from within fluid inlet cannula 426 toward secondary chamber 427, such as into and through porous vent 910, when patient puncture tip 438 is removed from the patient and is exposed to the external environment. In one particular embodiment, the total interior volume of the housing 412 is about 380 mm$^3$, with the flashback chamber 426 having a volume of about 30 mm$^3$, the secondary chamber 427 having a volume of about 300 mm$^3$, and the pores of the porous vent 910 representing a volume of about 50 mm$^3$.

Needle assembly 410 may be assembled as follows. Fluid inlet cannula 436 is positioned through first end 414 of housing 412 such that the open interior end 439 is positioned within an interior portion of housing 412 at first portion 419 and patient puncture tip 438 extends externally of first end 414. Fluid outlet cannula 452 is positioned within housing 412 through the opposite end, such that open internal end 464 is positioned within an interior portion of housing 412 at first portion 419 adjacent interior end 439 of fluid inlet cannula 436, with a slight gap therebetween, and with non-patient puncture tip extending externally of second end 416. Fluid inlet cannula 436 and fluid outlet cannula 452 may be affixed therein in any known manner, desirably through a medical grade adhesive.

In alternate embodiments including only a single cannula 470, such cannula 470 is affixed within housing 412 such that opening 472 is positioned within the interior of housing 412 at first portion 419, with patient puncture tip 438 extending externally of first end 414 and non-patient puncture tip 462 extending externally of second end 416.

Porous vent 910 is then inserted within housing 412 and positioned over fluid outlet cannula 454 (or over the single cannula 470), and element 428 is thereafter affixed to the second end 416, enclosing the interior of housing 412. Sealable sleeve 461 is then affixed over protrusion 429. As such, the interior of housing 412 is closed from the external environment, with the sole path for fluid communication between the interior of housing 412 and the external environment being provided through the patient puncture tip 438.

Needle assembly 410 assembled as such can be used in connection with a blood collection tube holder 800, as depicted in the embodiment shown in FIG. 12. Such assembly may be accomplished through the rear open end of blood collection tube holder 800, so that the entire needle assembly 410 is inserted to a portion where at least patient puncture tip 438 and at least a portion of inlet cannula 436 extend out through the front end of blood collection tube holder 800. In embodiments where second portion 421 of needle assembly 410 is radially larger than first portion 419, such an insertion and arrangement enables the secondary chamber 427 to be fully contained within the internal space within collection tube holder 800, and with flashback chamber 426 extending out from a front end thereof.

In use, needle assembly 410 may be provided with collection tube holder 800 attached thereto. Patient puncture tip 438 is inserted through the skin of a patient and into the patient's vasculature, desirably into a vein. Upon venipucture, a closed environment is achieved within housing 412, since housing 412 is an entirely closed structure, and since sealable sleeve 461 closes off the only outlet of housing 412 (i.e., fluid outlet cannula 452). The patient's blood pressure causes blood to flow through patient puncture tip 438, into fluid inlet cannula, 436, and out interior end 439 (or through opening 472 in the embodiment of FIG. 12), into flashback chamber 426 surrounding interior end 464 of outlet cannula 452. The transparent or translucent nature of housing **412** permits visualization of the blood within flashback chamber **426,** providing an indication that venipuncture is achieved.

Since the interior of housing **412** is a closed environment, the flow of blood into flashback chamber **426** causes air to be trapped within the housing interior, including within flashback chamber **426,** porous vent **910** and secondary chamber **427,** as well as within fluid outlet cannula **452,** causing such trapped air to be slightly pressurized therein. Flashback chamber **426** and secondary chamber **427** are configured through their size and dimensions such that the volumes thereof permit blood to flow into flashback chamber **426** at this initial venipucture, but the build up of air pressure within the pores of porous vent **910** and within secondary chamber **427** prevents blood from fully contacting porous vent **910,** and desirably prevents blood from even partially contacting porous vent **910** at the initial venipuncture.

After such initial venipuncture and flash visualization, a sample collection container having a negative pressure therein, such as an evacuated blood collection tube (not shown) as is commonly known in the art, is inserted within the tube holder **800.** The stopper (not shown) of such evacuated container contacts and displaces sealable sleeve **461,** causing non-patient puncture tip **462** to puncture through sealable sleeve **461** and through the stopper of the evacuated container. At this point, fluid communication is established between the non-patient puncture tip **462** and the interior of the evacuated collection container. The negative pressure within the evacuated collection container draws the blood that has collected within flashback chamber **426** into fluid outlet cannula **452** and into the evacuated collection container. Along with the blood within flashback chamber **426,** the negative pressure within the evacuated collection container will also draw at least a portion of the air out of the flashback chamber **426** and out of the secondary chamber **427** through the pores of porous vent 910, toward and into the evacuated collection container. In addition, the close proximity and alignment of fluid outlet cannula **452** and fluid inlet cannula 426 causes blood to be drawn from fluid inlet cannula **436** and from the patient, simultaneously with such air being drawn from the flashback chamber **426** and secondary chamber **427.**

Such drawing of air reduces the pressure within the flashback chamber **426** and the secondary chamber **427,** establishing a negative pressure therein with respect to the patient's bloodstream and with respect to the external environment. This negative pressure that has been established within the interior of **housing 412, and specifically within** flashback chamber **426** and secondary chamber **427,** draws additional blood from within fluid inlet cannula **436** and from the patient into flashback chamber **426,** with the blood contacting porous vent **910.** With such blood filling flashback chamber **426,** the blood fully contacts the surface of porous vent **910** that extends within flashback chamber **426,** and begins to fill the pores of porous vent **910.** Such filling of the pores of porous vent **910** that are directly at the interface of porous vent **910** and flashback chamber **426** closes off the porous vent from airflow therethrough, but does not fully act as a seal, in that the blood does not cause the material of the porous vent to swell or close off to air flow, but instead merely physically fills the voids within the porous vent. Moreover, since a portion of the air within secondary chamber **427** has been drawn out from secondary chamber **427,** secondary chamber **427** represents a closed chamber with a negative pressure therein relative to the external environment. Secondary chamber **427** will therefore continue to have a drawing effect on the blood within the pores of porous vent **910** and within flashback chamber **426** through the pores of porous vent **910** toward secondary chamber **427,** without releasing any air from the secondary chamber **427** in the opposite direction due to the pores of porous vent **910** at the interface of the flashback chamber **426** being filled with blood, thereby effectively preventing air flow through porous vent **910** due to the filled pores. The draw created by the negative pressure within secondary chamber **427** has a fluid resistance based on the blood filling the pores of porous vent **910** and based on the tortuous path created by the pores of porous vent **910,** and therefore is a gradual draw with reduced fluid movement.

At this point, the evacuated collection container and the secondary chamber **427** are both at a negative pressure with respect to the external environment (and with respect to the patient's bloodstream), and therefore both effect a draw from the fluid inlet cannula **436.** This mutual drawing effect may essentially establish an equilibrium within the flashback chamber **426,** such that the blood contained within the flashback chamber **426** is not drawn toward or into either the secondary chamber **427** through the pores of porous vent **910** or into the evacuated collection container through the fluid inlet cannula **436,** but instead essentially remains within flashback chamber **426** in a steady state. The negative pressure of the evacuated collection container draws blood directly from the patient through fluid inlet cannula **436,** due to the close proximity and alignment of fluid outlet cannula **452** and fluid inlet cannula **426,** as well as due to the equilibrium established within flashback chamber **426** (based on the opposite draw forces between the evacuated collection container and the evacuated secondary chamber **427).** The continual draw of blood into the evacuated collection container gradually causes the pressure within the collection container to increase.

Once the evacuated collection container is filled with the desired amount of blood, the container is removed from the non-patient puncture tip **462,** thereby releasing the fluid communication between the non-patient puncture tip **462** and the evacuated collection container, with sealable sleeve **461** then covering and closing off non-patient puncture tip **462.** Absent such draw from the negative pressure of the evacuated collection tube, the negative pressure within the secondary chamber **427** effects a slight draw on the blood within flashback chamber **426** through the pores of porous vent **910.** Such draw, however, is slow and gradual, due to the tortuous path of blood flow through the pores of porous vent **910.**

Additional evacuated collection containers can thereafter be inserted into tube holder **800** and used for sample collection through non-patient puncture tip **462** as described above, by placing a second evacuated collection container within the holder **800** and establishing fluid communication between the non-patient puncture tip **462** and the interior of the evacuated collection container by puncturing the stopper, as discussed. In such further sampling, the evacuated collection container and the secondary chamber **427** are both at a negative pressure, and therefore both effect a draw from the fluid inlet cannula. As above, this effect essentially establishes an equilibrium within the flashback chamber **426,** thereby preventing the blood contained within the flashback chamber **426** from being drawn toward or into either the secondary chamber **427** (through the porous vent **910).** The negative pressure of the evacuated collection container draws blood directly from the patient through fluid inlet cannula **436** as discussed above, due to the close proximity and alignment of fluid outlet cannula **452** and fluid inlet cannula **426.** Once any such additional evacuated collection containers are filled with the desired amount of blood, the container is removed from the non-patient puncture tip **462,** thereby releasing the fluid communication between the non-patient puncture tip **462** and the evacuated collection container, with sealable sleeve **461** then covering and closing off non-patient puncture tip **462.**

Once all of the desired blood samples have been drawn in this manner, patient puncture tip **438** is removed from the vasculature of the patient (i.e. from the bloodstream), thereby exposing the opening of patient puncture tip **438** to the external environment. Since the sole communication path between the housing interior and the external environment is through patient puncture tip **438,** the negative pressure established within secondary chamber **427** relative to the external environment will affect a gradual draw on the blood contained within flashback chamber **426** and within fluid inlet cannula **436** toward and through porous vent **910.** Such drawing effect will displace and move any blood contained within fluid inlet cannula **436** away from patient puncture tip **438,** toward secondary chamber **427,** thereby preventing any blood from leaking from patient puncture tip **438** out of fluid inlet cannula **436.** Such negative pressure within secondary chamber **427** may continue to have a gradual drawing effect through the porous vent **910** for a prolonged period of time after removal of patient puncture tip **438** from the patient, and may draw all of the remaining blood contained within fluid inlet cannula **436** and flashback chamber **426** through porous vent **910** and/or into secondary chamber **427.** Needle assembly **410** can then be properly disposed of in known manner.

**Claims**

**1.** A needle assembly comprising:

a housing (412) defining a housing interior;
a cannula (436, 452) having a patient puncture tip (438) extending from a first end (414) of the housing (412);
a non-patient puncture tip (462) extending from a second end (416) of the housing (412), the non-patient puncture tip (462) and the patient puncture tip (438) being in fluid communication with each other through the cannula (436, 452), wherein the sole communication path between the housing interior and the external environment is via the patient puncture tip (438); and
a porous vent (910) positioned within the housing interior to separate the housing interior into a first chamber (426) and a second chamber (427) with the cannula (436, 452) being in fluid communication with the first chamber (426), the porous vent (910) including pores for passage of blood therethrough from the first chamber (426) to the second chamber (427),
**characterized in that**
the first chamber (426) and the second chamber (427) and the porous vent (910) are configured such that upon insertion of the patient needle tip (438) into a patient blood flows through said cannula (436, 452) and into the first chamber (426) without sealing the porous vent (910), and
upon application of an evacuated container to said non-patient puncture tip (462), blood is drawn from said first chamber (426) and air is drawn from said second chamber (427) through the porous vent (910), thereby establishing a negative pressure gradient within said second chamber (427) with respect to an external environment of the needle assembly.

**2.** The needle assembly of claim 1, wherein the cannula (436, 452) includes a first end comprising the patient puncture tip (438) and a second end comprising the non-patient puncture tip (462), with an opening (472) between the first end and the second end providing fluid communication between the cannula (436, 452) and the first chamber (426) of the housing (412).

**3.** The needle assembly of claim 1, wherein the cannula (436, 452) comprises a first cannula (436) having a patient puncture tip (438), and wherein the needle assembly further comprises a second cannula (452) including the non-patient puncture tip (462), the first cannula (436) and the second cannula (452) being substantially axially aligned and separated by a gap in fluid communication with the first chamber (426) of the housing (412).

**4.** The needle assembly of claim 1, further comprising a sleeve (461) extending about the non-patient puncture tip (462).

**5.** The needle assembly of claim 1, configured such that upon at least a portion of air being drawn from said first chamber (426) and said second chamber (427), said second chamber (427) draws blood from the first chamber (426) through the porous vent (910).

6. The needle assembly of claim 1, wherein at least a portion of said housing (412) forming said first chamber (426) is formed from a transparent or translucent material.

7. The needle assembly of claim 1, wherein the first end of the housing comprises an elongate longitudinal first portion (419) having a first diameter and the second end of the housing comprises a second portion (421) having a second diameter larger than the first diameter of the first portion (419).

8. The needle assembly of claim 7, wherein the porous vent (910) is positioned within the housing interior between the first portion (419) having a first diameter and the second portion (421) having a second diameter or wherein the porous vent (910) is positioned within the housing interior at a location spanning the transition between the first diameter of the first position (419) and the second diameter of the second position (421).

9. The needle assembly of claim 1, wherein the housing interior has a total volume of from 300 to 400 mm$^3$, and the first chamber (426) has a volume from 5 percent to 20 percent of the total volume of the housing interior.

10. A method of preventing leakage of blood from a needle assembly comprising:

   a) receiving blood through a patient puncture tip (438) and into a first chamber (426) of a needle assembly, the needle assembly comprising:

   i) a needle housing (412) defining a housing interior;
   ii) a cannula (436, 452) having the patient puncture tip (438) extending from a first end (414) of the needle housing (412);
   iii) a non-patient puncture tip (462) extending from a second end (416) of the needle housing (412), the non-patient puncture tip (462) and the patient puncture tip (438) being in fluid communication with each other through the cannula (436, 452); and
   iv) a porous vent (910) positioned within the housing interior and separating the housing interior into a first chamber (426) and a second chamber (427), with the cannula (436, 452) being in fluid communication with the first chamber (426) such that the sole communication path between the housing interior and the external environment is via the patient puncture tip (438), the porous vent (910) including pores for passage of blood therethrough from the first chamber (426) into the second chamber (427);

   **characterized by**
   b) establishing fluid communication between the non-patient puncture tip (462) and an evacuated collection container, such that blood contained within the first chamber (426) is drawn into the evacuated collection container and air is drawn out of the second chamber (427) through the porous vent (910), thereby establishing a negative pressure gradient within the second chamber (427) relative to the external environment of the needle assembly such that blood flows through the cannula (436) into the first chamber (426) and contacts the porous vent (910); and
   c) drawing blood through the pores of the porous vent (910) toward the second chamber (427) based upon the negative pressure gradient established within the second chamber (427) such that any blood contained within the cannula (436) is displaced away from the patient puncture tip (438) and toward the second chamber (427).

11. The method of claim 10, including a further step after step b) and prior to step c) comprising releasing the fluid communication between the non-patient puncture tip (462) and the evacuated collection container.

12. The method of claim 11, wherein the step of releasing the fluid communication comprises removing the evacuated collection container from the non-patient puncture tip (462) and sealing the non-patient puncture tip (462) from the external environment.

13. The method of claim 12, wherein the needle assembly comprises a sealable sleeve (461) extending about the non-patient puncture tip (462), said sealable sleeve (461) being displaceable so as to permit the fluid communication between the evacuated collection container and the non-patient puncture tip (462) in step b), and so as to reseal upon removal of the evacuated collection container from the non-patient puncture tip (462).

14. The method of claim 11, including a further step after said step of releasing the fluid communication between the non-patient puncture tip (462) and the evacuated collection container, comprising establishing fluid communication between the non-patient puncture tip (462) and a second evacuated collection container, such that blood is drawn through the patient puncture tip (438) and through the cannula (436, 452) into the second evacuated collection container, followed by releasing the fluid communication between the non-patient puncture tip (462) and the second evacuated collection container.

## Patentansprüche

1. Nadelanordnung mit:

   einem Gehäuse (412), das einen Gehäuseinnenraum begrenzt;
   einer Kanüle (436, 452) mit einer Patientenpunktionsspitze (438), die sich von einem ersten Ende (414) des Gehäuses (412) aus erstreckt;
   einer Nicht-Patientenpunktionsspitze (462), die sich von einem zweiten Ende (416) des Gehäuses (412) erstreckt, wobei die Nicht-Patientenpunktionsspitze (462) und die Patientenpunktionsspitze (438) durch die Kanüle (436, 452) miteinander in Fluidverbindung stehen, wobei der einzige Verbindungsweg zwischen dem Gehäuseinnenraum und der äußeren Umgebung über die Patientenpunktionsspitze (438) besteht; und
   einer porösen Lüftungseinrichtung (910), die in dem Gehäuseinnenraum angeordnet ist, um den Gehäuseinnenraum in eine erste Kammer (426) und eine zweite Kammer (427) zu trennen, wobei die Kanüle (436, 452) mit der ersten Kammer (426) in Fluidverbindung steht, wobei die poröse Lüftungseinrichtung (910) Poren für den Durchtritt von Blut aus der ersten Kammer (426) in die zweite Kammer (427) aufweist,
   **dadurch gekennzeichnet, dass**
   die erste Kammer (426) und die zweite Kammer (427) und die poröse Lüftungseinrichtung (910) derart ausgebildet sind, dass beim Einführen der Patientennadelspitze (438) in einen Patienten Blut durch die Kanüle (436, 452) und in die erste Kammer (426) strömt, ohne die poröse Lüftungseinrichtung (910) abzudichten, und dass beim Ansetzen eines evakuierten Behälters an die Nicht-Patientenpunktionsspitze (462) Blut aus der ersten Kammer (426) und Luft aus der zweiten Kammer (427) durch die poröse Lüftungseinrichtung (910) gesogen wird, wodurch in der zweiten Kammer (427) ein negativer Druckgradient in Bezug auf die äußere Umgebung der Nadelanordnung erzeugt wird.

2. Nadelanordnung nach Anspruch 1, bei welcher die Kanüle (436, 452) ein erstes Ende, das die Patientenpunktionsspitze (438) aufweist, und ein zweites Ende aufweist, das die Nicht-Patientenpunktionsspitze (462) aufweist, wobei eine Öffnung (472) zwischen dem ersten Ende und dem zweiten Ende eine Fluidverbindung zwischen der Kanüle (436, 452) und der ersten Kammer (426) des Gehäuses (412) bewirkt.

3. Nadelanordnung nach Anspruch 1, bei welcher die Kanüle (436, 452) eine erste Kanüle (436) mit einer Patientenpunktionsspitze (438) aufweist, und wobei die Nadelanordnung ferner eine zweite Kanüle (452) mit der Nicht-Patientenpunktionsspitze (462) aufweist, wobei die erste Kanüle (436) und die zweite Kanüle (452) im Wesentlichen axial ausgerichtet sind und durch einen Spalt voneinander getrennt sind, der in Fluidverbindung mit der ersten Kammer (426) des Gehäuses (412) steht.

4. Nadelanordnung nach Anspruch 1, ferner mit einer Hülse (461), die sich um die Nicht-Patientenpunktionsspitze (462) erstreckt.

5. Nadelanordnung nach Anspruch 1, welche derart ausgebildet ist, dass sobald zumindest ein Teil der Luft aus der ersten Kammer (426) und der zweiten Kammer (427) gesogen ist, die zweite Kammer (427) Blut aus der ersten Kammer (426) durch die poröse Lüftungseinrichtung (910) saugt.

6. Nadelanordnung nach Anspruch 1, bei welcher zumindest ein Teil des Gehäuses (412), der die erste Kammer (426) bildet, aus einem transparenten oder transluzenten Material gebildet ist.

7. Nadelanordnung nach Anspruch 1, bei welcher das erste Ende des Gehäuses einen langgestreckten ersten Bereich (419) mit einem ersten Durchmesser und das zweite Ende des Gehäuses einen zweiten Bereich (421) mit einem zweiten Durchmesser aufweist, der größer als der erste Bereich (419) ist.

8. Nadelanordnung nach Anspruch 7, bei welcher die poröse Lüftungseinrichtung (910) in dem Gehäuseinnenraum zwischen dem einen ersten Durchmesser aufweisenden ersten Bereich (419) und dem einen zweiten Durchmesser aufweisenden zweiten Bereich (421) angeordnet ist, oder wobei die poröse Lüftungseinrichtung (910) in dem Gehäuseinnenraum an einer Stelle angeordnet ist, welche den Übergang zwischen dem ersten Durchmesser des ersten Bereichs (419) und dem zweiten Durchmesser des zweiten Bereichs (421) überspannt.

9. Nadelanordnung nach Anspruch 1, bei welcher der Gehäuseinnenraum ein Gesamtvolumen zwischen 300 und 400 mm$^3$ aufweist, und wobei die erste Kammer (426) ein Volumen zwischen 5 Prozent und 20 Prozent des Gesamtvolumens des Gehäuseinnenraums aufweist.

10. Verfahren zum Verhindern des Austretens von Blut aus einer Nadelanordnung, mit den folgenden Schritten:

    a) Aufnehmen von Blut durch eine Patientenpunktionsspitze (438) und in eine erste Kammer (426) einer Nadelanordnung, wobei die Nadelanordnung aufweist:

i) ein Nadelgehäuse (412), das einen Gehäuseinnenraum begrenzt;
ii) eine Kanüle (436, 452) mit einer Patientenpunktionsspitze (438), die sich von einem ersten Ende (414) des Nadelgehäuses (412) aus erstreckt;
iii) eine Nicht-Patientenpunktionsspitze (462), die sich von einem zweiten Ende (416) des Nadelgehäuses (412) erstreckt, wobei die Nicht-Patientenpunktionsspitze (462) und die Patientenpunktionsspitze (438) durch die Kanüle (436, 452) miteinander in Fluidverbindung stehen; und
iv) einer in dem Gehäuseinnenraum angeordneten porösen Lüftungseinrichtung (910), die den Gehäuseinnenraum in eine erste Kammer (426) und eine zweite Kammer (427) trennt, wobei die Kanüle (436, 452) mit der ersten Kammer (426) derart in Fluidverbindung steht, dass der einzige Verbindungsweg zwischen dem Gehäuseinnenraum und der äußeren Umgebung über die Patientenpunktionsspitze (438) besteht, wobei die poröse Lüftungseinrichtung (910) Poren für den Durchtritt von Blut aus der ersten Kammer (426) in die zweite Kammer (427) aufweist,

**gekennzeichnet durch**

b) das Herstellen einer Fluidverbindung zwischen der Nicht-Patientenpunktionsspitze (462) und einem evakuierten Sammelbehälter derart, dass über die poröse Lüftungseinrichtung (910) in der ersten Kammer (426) enthaltenes Blut in den evakuierten Sammelbehälter und Luft aus der zweiten Kammer (427) gesogen wird, wodurch in der zweiten Kammer (427) ein negativer Druckgradient in Bezug auf die äußere Umgebung der Nadel derart erzeugt wird, dass Blut **durch** die Kanüle (436) in die erste Kammer (426) fließt und in Kontakt mit der porösen Lüftungseinrichtung (910) gelangt; und
c) das Ziehen von Blut **durch** die Poren der porösen Lüftungseinrichtung (910) in Richtung der zweiten Kammer (427) aufgrund des in der zweiten Kammer (427) erzeugten negativen Druckgradienten derart, dass jegliches in der Kanüle (436) enthaltenes Blut von der Patientenpunktionsspitze (438) weg und in Richtung der zweiten Kammer (427) bewegt wird.

**11.** Verfahren nach Anspruch 10, mit einem weiteren Schritt nach dem Schritt b) und vor dem Schritt c), welcher das Aufheben der Fluidverbindung zwischen der Nicht-Patientenpunktionsspitze (462) und dem evakuierten Sammelbehälter aufweist.

**12.** Verfahren nach Anspruch 11, bei welchem der Schritt des Aufhebens der Fluidverbindung das Entfernen des evakuierten Sammelbehälters von der Nicht-Patientenpunktionsspitze (462) und das Abdichten der Nicht-Patientenpunktionsspitze (462) gegenüber der äußeren Umgebung aufweist.

**13.** Verfahren nach Anspruch 12, bei welchem die Nadelanordnung eine abdichtende, sich über die Nicht-Patientenpunktionsspitze (462) erstreckende Hülse aufweist, wobei die abdichtbare Hülse (461) bewegbar ist, um die Fluidverbindung zwischen der evakuierten Sammelkammer und der Nicht-Patientenpunktionsspitze (462) in Schritt b) zu ermöglichen, und um beim Entfernen des evakuierten Sammelbehälters von der Nicht-Patientenpunktionsspitze (462) wieder abzudichten. (Bitte im Englischen prüfen)

**14.** Verfahren nach Anspruch 11, mit einem weiteren Schritt nach dem Schritt des Aufhebens der Fluidverbindung zwischen der Nicht-Patientenpunktionsspitze (462) und dem evakuierten Sammelbehälter, mit dem Herstellen einer Fluidverbindung zwischen der Nicht-Patientenpunktionsspitze (462) und einem zweiten evakuierten Sammelbehälter derart, dass Blut durch die Patientenpunktionsspitze (438) und durch die Kanüle (436, 452) in den zweiten evakuierten Sammelbehälter gesogen wird, wonach die Fluidverbindung zwischen der Nicht-Patientenpunktionsspitze (462) und dem zweiten evakuierten Sammelbehälter aufgehoben wird.

**Revendications**

**1.** Ensemble d'aiguille comprenant :

un logement (412) définissant une partie intérieure de logement ;
une canule (436, 452) ayant une pointe de ponction côté patient (438) s'étendant à partir d'une première extrémité (414) du logement (412) ;
une pointe de ponction côté opposé au patient (462) s'étendant à partir d'une deuxième extrémité (416) du logement (412), la pointe de ponction côté opposé au patient (462) et la pointe de ponction côté patient (438) étant en communication fluidique l'une avec l'autre à travers la canule (436, 452), où le seul chemin de communication entre la partie intérieure de logement et l'environnement extérieur est assuré par la pointe de ponction côté patient (438) ; et
un évent poreux (910) positionné dans la partie intérieure de logement pour séparer la partie intérieure de logement en une première chambre (426) et une deuxième chambre (427) avec la canule (436, 452) étant en communication fluidique avec la première chambre (426), l'évent poreux (910) comportant des pores pour le passage du sang à travers ceux de la première chambre (426) à la deuxième chambre (427), **caractérisé en ce que**

la première chambre (426) et la deuxième chambre (427) et l'évent poreux (910) sont configurés de sorte que, lors de l'insertion de la pointe d'aiguille côté patient (438) dans un patient, le sang s'écoule à travers ladite canule (436, 452) et dans la première chambre (426) sans sceller l'évent poreux (910), et lors de l'application d'un récipient sous vide à ladite pointe de ponction côté opposé au patient (462), le sang est aspiré de ladite première chambre (426) et l'air est aspiré de ladite deuxième chambre (427) à travers l'évent poreux (910), établissant ainsi un gradient de pression négative dans ladite deuxième chambre (427) par rapport à un environnement extérieur de l'ensemble d'aiguille.

2. Ensemble d'aiguille de la revendication 1, dans lequel la canule (436, 452) comporte une première extrémité comprenant la pointe de ponction côté patient (438) et une deuxième extrémité comprenant la pointe de ponction côté opposé au patient (462), avec une ouverture (472) entre la première extrémité et la deuxième extrémité assurant la communication fluidique entre la canule (436, 452) et la première chambre (426) du logement (412).

3. Ensemble d'aiguilles de la revendication 1, dans lequel la canule (436, 452) comprend une première canule (436) ayant une pointe de ponction côté patient (438), et où l'ensemble d'aiguille comprend en outre une deuxième canule (452) comportant la pointe de ponction côté opposé au patient (462), la première canule (436) et la deuxième canule (452) étant alignées de manière sensiblement axiale et séparées par un espace en communication fluidique avec la première chambre (426) du logement (412).

4. Ensemble d'aiguille de la revendication 1, comprenant en outre un manchon (461) s'étendant autour de la pointe de ponction côté opposé au patient (462).

5. Ensemble d'aiguille de la revendication 1, configuré de sorte que, lors de l'aspiration d'au moins une partie d'air de ladite première chambre (426) et de ladite deuxième chambre (427), ladite deuxième chambre (427) aspire le sang de la première chambre (426) à travers l'évent poreux (910).

6. Ensemble d'aiguille de la revendication 1, dans lequel au moins une partie dudit logement (412) formant ladite première chambre (426) est formée d'un matériau transparent ou translucide.

7. Ensemble d'aiguille de la revendication 1, dans lequel la première extrémité du logement comprend une première partie longitudinale allongée (419) ayant un premier diamètre et la deuxième extrémité du logement comprend une deuxième partie (421) ayant un deuxième diamètre supérieur à celui de la première partie (419).

8. Ensemble d'aiguille de la revendication 7, dans lequel l'évent poreux (910) est positionné dans la partie intérieure de logement entre la première partie (419) ayant un premier diamètre et la deuxième partie (421) ayant un deuxième diamètre, ou où l'évent poreux (910) est positionné dans la partie intérieure de logement à un emplacement couvrant la transition entre le premier diamètre de la première position (419) et le deuxième diamètre de la deuxième position (421).

9. Ensemble d'aiguille de la revendication 1, dans lequel la partie intérieure de logement a un volume total allant de 300 à 400 mm$^3$, et la première chambre (426) a un volume allant de 5 pour cent à 20 pour cent du volume total de la partie intérieure de logement.

10. Procédé pour empêcher une fuite de sang à partir d'un ensemble d'aiguille comprenant le fait :

a) de recevoir le sang à travers une pointe de ponction côté patient (438) et dans une première chambre (426) d'un ensemble d'aiguille, l'ensemble d'aiguille comprenant :

i) un logement d'aiguille (412) définissant une partie intérieure de logement ;
ii) une canule (436, 452) ayant la pointe de ponction côté patient (438) s'étendant à partir d'une première extrémité (414) du logement d'aiguille (412) ;
iii) une pointe de ponction côté opposé au patient (462) s'étendant à partir d'une deuxième extrémité (416) du logement d'aiguille (412), la pointe de ponction côté opposé au patient (462) et la pointe de ponction côté patient (438) étant en communication fluidique l'une avec l'autre à travers la canule (436, 452) ; et
iv) un évent poreux (910) positionné dans la partie intérieure de logement et séparant la partie intérieure de logement en une première chambre (426) et une deuxième chambre (427), avec la canule (436, 452) étant en communication fluidique avec la première chambre (426) de sorte que le seul chemin de communication entre la partie intérieure de logement et l'environnement extérieur soit assuré par la pointe de ponction côté patient (438), l'évent poreux (910) comportant des pores pour le passage du sang à travers ceux-ci à partir de la première chambre (426) dans la deuxième chambre (427) ;

**caractérisé par** le fait

b) d'établir une communication fluidique entre la pointe de ponction côté opposé au patient (462) et un récipient de prélèvement sous vide, de sorte que le sang contenu dans la première chambre (426) est aspiré dans le récipient de prélèvement sous vide et l'air est retiré de la deuxième chambre (427) à travers l'évent poreux (910), établissant ainsi un gradient de pression négative dans la deuxième chambre (427) par rapport à l'environnement extérieur de l'ensemble d'aiguille de sorte que le sang s'écoule à travers la canule (436) dans la première chambre (426) et entre en contact avec l'évent poreux (910) ; et

c) d'aspirer le sang à travers les pores de l'évent poreux (910) vers la deuxième chambre (427) sur la base du gradient de pression négative établi dans la deuxième chambre (427) de sorte que tout sang contenu dans la canule (436) soit éloigné de la pointe de ponction côté patient (438) et vers la deuxième chambre (427).

11. Procédé de la revendication 10, comportant une étape supplémentaire après l'étape b) et avant l'étape c) comprenant le fait de couper la communication fluidique entre la pointe de ponction côté opposé au patient (462) et le récipient de prélèvement sous vide.

12. Procédé de la revendication 11, dans lequel l'étape de coupure de la communication fluidique comprend le fait de retirer le récipient de prélèvement sous vide de la pointe de ponction côté opposé au patient (462) et d'assurer l'étanchéité de la pointe de ponction côté opposé au patient (462) par rapport à l'environnement extérieur.

13. Procédé de la revendication 12, dans lequel l'ensemble d'aiguille comprend un manchon scellable s'étendant sur la pointe de ponction côté opposé au patient (462), ledit manchon scellable (461) pouvant être déplacé de manière à permettre la communication fluidique entre le récipient de prélèvement sous vide et la pointe de ponction côté opposé au patient (462) à l'étape b), et de manière à être rescellé lors du retrait du récipient de prélèvement sous vide de la pointe de ponction côté opposé au patient (462).

14. Procédé de la revendication 11, comportant une étape supplémentaire après ladite étape de coupure de la communication fluidique entre la pointe de ponction côté opposé au patient (462) et le récipient de prélèvement sous vide, comprenant l'établissement d'une communication fluidique entre la pointe de ponction côté opposé au patient (462) et un deuxième récipient de prélèvement sous vide, de sorte que le sang soit aspiré à travers la pointe de ponction côté patient (438) et à travers la canule (436, 452) dans le deuxième récipient de prélèvement sous vide, suivi de la coupure de la communication fluidique entre la pointe de ponction côté opposé au patient (462) et le deuxième récipient de prélèvement sous vide.

FIG.1

FIG.2

FIG.3

FIG.4

FIG.5

EP 2 254 472 B1

FIG.6

FIG.7

EP 2 254 472 B1

410

430

461

436

438

FIG.8

436

410

461

430

FIG.9

FIG.10

EP 2 254 472 B1

FIG.11A

FIG.11B

FIG.12A

**FIG.12B**

EP 2 254 472 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5222502 A
- US 5303713 A
- WO 2006022716 A1
- US 20060036219 A1